# EUROPEAN PATENT SPECIFICATION

(11) **EP 2 413 981 B1**
(45) Date of publication and mention of the grant of the patent: **10.07.2013**
(21) Application number: 10723433.8
(22) Date of filing: 31.03.2010
(51) Int. Cl.: A61L 15/26, A61L 15/42, A61L 27/18, A61L 27/56, A61L 31/06, A61L 31/14, C08J 9/28

(54) **THICK FOAMS FOR BIOMEDICAL APPLICATIONS AND METHODS OF MAKING**
DICKE SCHAUMSTOFFE FÜR BIOMEDIZINISCHE ANWENDUNGEN UND HERSTELLUNGSVERFAHREN
MOUSSES ÉPAISSES POUR APPLICATIONS BIOMÉDICALES ET PROCÉDÉS DE FABRICATION

(30) Priority: 31.03.2009 US 415260
(43) Date of publication of application: 08.02.2012
(73) Proprietor: Advanced Technologies and Regenerative Medicine, LLC, Raynham, MA 02767 (US)
(72) Inventor: SHETTY, Dhanuraj, S., Jersey City, NJ 07302 (US); CHUN, Iksoo, Princeton NJ 08540 (US); VYAKARNAM, Murty, N., Bridgewater, NJ 08807 (US); HYLAND, Thomas, Patrick, Blairhill Coatbridge ML5 1NR (GB)
(74) Representative: James, Anthony Christopher W.P.
(86) International application number: PCT/US2010/029293
(87) International publication number: WO 2010/117824

(56) References cited:
- EP-A1- 1 216 717
- EP-A1- 1 264 607
- EP-A1- 1 273 615
- WO-A1-01/02033
- WO-A2-2009/018126
- WO-A2-2009/029087
- US-A- 5 516 395

## Description

### FIELD OF THE INVENTION

The invention relates generally to the field of tissue repair and regeneration. Specifically, the invention relates to methods of making open cell porous biocompatible foams for use with tissue repair and regeneration.

### BACKGROUND OF THE INVENTION

Open cell porous biocompatible foams have been recognized to have significant potential for use in repair and regeneration of tissue. Among the potential uses for such foams are drug delivery systems, neural regeneration, vascular replacements and artificial bone templates. Specific areas of immediate significance include the use of biodegradable microcellular foams for bone and cartilage regeneration applications as well as the use of microcellular foams for organ generation. Prior attempts in tissue repair and regeneration have utilized amorphous biocompatible foams as a porous plug to fill voids in bone.

For example it is known to have porous open cell foams of polyhydroxy acids having pore sizes ranging from about 10 to about 200 micrometers for the in-growth of blood vessels and cells. Such foams can be reinforced with fibers, yarns, and braids, knitted fabrics, scrims and the like. The foams may consist of a variety of polyhydroxy acid polymers and copolymers such as poly-L-lactide, poly-DL-lactide, polyglycolide, and polydioxanone. Also known in this are three-dimensional interconnected open cell porous foams that have a gradient in composition and/or microstructure through one or more directions. Another example of known foams are three-dimensional laminated foams made in the following manner. A porous membrane is initially prepared by drying a polymer solution containing leachable salt crystals. A three dimensional structure is then obtained by laminating several membranes together which are then cut using a contour drawing of the desired shape. However, this process is quite cumbersome and long.

Conventional lyophilization lends itself to many advantages when processing thermally sensitive polymers, and is one method of manufacturing polymer foams. Further, it lends itself to aseptic processing methodologies for biodegradable applications especially when using combinations of polymers with drugs or other bioactive agents such as growth factors, proteins etc. A conventional lyophlization process is conducted in the following manner: A polymer solution is prepared with a known concentration. After the solution is prepared, it is poured into a mold. The mold containing the polymer solution is then placed onto the freeze dryer shelf that is run through the complete lyophilization cycle that includes the freezing step followed by the drying step. The technology has been limited, however to preparing thin foams having a thickness of less than about 1cm and having a uniform porosity along the cross section of the foam. Attempts to prepare thick foams (greater than 1cm in thickness) have failed to produce foams with uniform porosity and morphology throughout the thickness of the foam, and such processes are time consuming having often required process times of more than 3 days to process one foam. Making uniform porous foams is difficult when using the two main traditional methods for making foams, namely low temperature freeze-drying (i.e., lyophilization) and salt leaching.

A conventional salt leaching process is conducted in the following manner: Salt particulates are prepared by sieving. The desirable size of the salt particulates are controlled by the sieving. Polymer solutions are prepared by dissolving different amounts and types of polymers in solvent (e.g. methylene chloride or chloroform) Sieved salt particulates are added to the polymer solution, and the dispersion is gently vortexed. The solution is poured into a mold. Subsequently, the mold with dispersion is pressed by pressure apparatus. The formed samples are taken out of the mold. Samples are dissolved for a desirable time (48h) in deionised water. Salt-removed samples are freeze dried for a desirable time (about 48h) at low temperature. The scaffolds are dried in an oven at 25°C for 1 week to remove the residual solvent. One limitation of salt leaching is that it is often difficult to form small micropores with salt and it requires a high salt loading to achieve interpore channeling to produce continuous microporous foams.

WO-A-0102033, EP-A-1264607, WO-A-2009018126 and EP-A-1216717 describe conventional lyophilisation (freeze-drying) methods for making microporous foams of biodegradable polymers.

WO-A-2009029087 describes methods for making superporous hydrogel foams by controlled foaming of during polymerization of the hydrogel monomers.

EP-A-1273615 describes methods of making microporous foams having directional pores by freeze-drying, wherein the freezing step is performed with a temperature gradient to achieve directional freezing resulting in directional pores in the product.

US-A-5516395 describes a process for the production of continuous collagen foam tapes including a conventional freeze-drying step.

There is a need in this art for novel processes for making high quality thick foams from biodegradable polymer having uniform structures.

### SUMMARY OF THE INVENTION

In a first aspect, the present invention provides a method of making a polymer foam member having a thickness of greater than 1 cm, comprising the steps of: providing a thermoreversible polymer solution, said solution comprising a biocompatible, biodegradable polymer and a solvent; placing said solution in a mold; cooling the solution in said mold to a precool temperature and holding the solution at said precool temperature for from 360 minutes to 1440 minutes until the solution gels; followed by removing the solvent by lyophilization to yield said foam member having a thickness of greater than 1 cm and inter-connected pores.

Another aspect of the present invention is a method of making a medical device comprising the steps of: making a foam member by a method according to the present invention, and cutting the foam member into a medical device.

These and other aspects and advantages of the present invention will become more apparent from the following description and drawings.

### BRIEF DESCRIPTION OF THE DRAWINGS

FIG 1: is a SEM image of a bottom cross- section of a thick (2.5 cm) polymer foam scaffold manufactured by the novel process of the present invention.
FIG 2: is a SEM image of a middle cross- section of a 2.5 cm foam scaffold.
FIG 3: is a SEM image of a top cross-section of a thick foam scaffold manufactured by the method of the present invention.

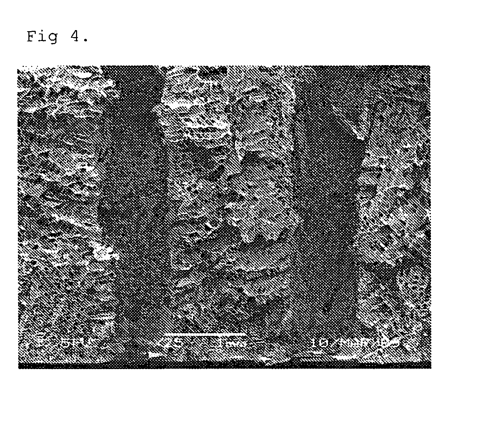

FIG 4. is a SEM image of a thick foam having channels manufactured by the process of the present invention.

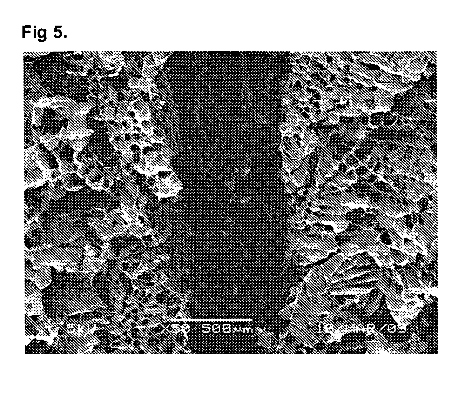

FIG 5. is a SEM image of a foam having channels manufactured by the novel process of the present invention.

### DETAILED DESCRIPTION OF THE INVENTION

The novel method of the present invention provides for making thick biocompatible, biodegradable foams that have inter-connected pores and further have a uniform morphological structure is disclosed. The term inter-connected pores is defined to have its conventional meaning as otherwise expanded herein, specifically where the cells are open cell structures that are interconnected with their neighboring cells that provide pathways for cells migration and nutrient transfer. The term uniform morphological structure is defined to have its conventional meaning as otherwise expanded herein, specifically the pore size ranges are uniform through the thickness of the scaffold.

The thick foams are prepared in accordance with the novel method of the present invention as defined in claim 1 by providing a thermoreversible polymer solution, pouring the solution into a mold, placing the mold on a precooled shelf in a lyophilizer for a sufficient period of time to cause the solution to gel, and removing the solvent from the gelled thermoreversible polymer solution by lyophilization, thereby providing a thick polymer foam.

For the purposes of this invention, "thick" is defined as greater than 1cm.

A thermoreversible polymer solution, for the purposes of this invention is defined as a polymer solution that will transition between a liquid and a gel depending upon the temperature of the solution. The process of gelation, which transforms a liquid into a gel, begins with a change in temperature, such as a decrease in temperature that favors the formation of a gel. The liquid to gel transition (and vice versa) is thermoreversible, such that a subsequent increase in temperature results in the gel becoming a liquid. Gel is defined as a continuous solid network enveloped in a continuous liquid phase. The gel/liquid transition temperature is a function of polymer concentration and solvent interaction.

The thermoreversible polymer solution is prepared by dissolving one or more biocompatible, biodegradable polymers in a suitable solvent, such as 1,4-dioxane. The polymer is present in the solution in the amount of typically about 0.5 to about 10 weight percent. In another embodiment the polymer is present in the solution in the amount of about 2 to about 6 weight percent. In yet another embodiment, the polymer is present in the solution in the amount of about 5 weight percent. Other concentrations of polymer in solution may be utilized depending upon the maximum concentration that may be made by using the solvent. For e.g. with 1,4-dioxane the maximum achievable concentration is 15% by weight of the polymer. The polymer is dissolved in the 1,4-dioxane at a sufficiently effective temperature to dissolve the polymer, for example, about 60°C, and preferably with agitation such as stirring. The solution is preferably filtered prior to pouring into a mold for lyophilization.

Examples of suitable biocompatible, biodegradable polymers useful to manufacture the thick foams of the present invention include polymers selected from the group consisting of aliphatic polyesters, poly(amino acids), copoly(ether-esters), polyalkylenes oxalates, polyamides, poly(iminocarbonates), polyorthoesters, polyoxaesters, polyamidoesters, polyoxaesters containing amine groups, poly(anhydrides), polyphosphazenes, biomolecules and blends thereof. For the purpose of this invention aliphatic polyesters include but are not limited to homopolymers and copolymers of lactide (which includes lactic acid, d-, 1- and meso lactide), glycolide (including glycolic acid), epsilon -caprolactone, p-dioxanone (1,4-dioxan-2-one), trimethylene carbonate (1, 3-dioxan-2-one), alkyl derivatives of trimethylene carbonate and blends thereof. In one embodiment aliphatic polyester is a copolymer of lactide and glycolide. In another embodiment, the aliphatic polyester is a copolymer of lactide and glycolide having a molar ratio of about 95:5.

One preferred class of aliphatic polyester polymers are elastomeric copolymers. For the purpose of this invention elastomeric copolymers are defined as a materials that at room temperature can be stretched repeatedly to at least about twice its original length and upon immediate release of stress, will return to approximately its original length. Suitable biodegradable, biocompatible elastomers include but are not limited to elastomeric copolymers of epsilon-caprolactone and glycolide (preferably having a mole ratio of epsilon-caprolactone to glycolide of from about 30:70 to about 70:30, preferably 35:65 to about 65:35, and more preferably 35:65 to 45:55); elastomeric copolymers of epsilon- caprolactone and lactide, including L-lactide, D-lactide blends thereof or lactic acid copolymers (preferably having a mole ratio of epsilon-caprolactone to lactide of from about 35:65 to about 65:35 and more preferably 30:70 to 45:55) elastomeric copolymers of p-dioxanone (1,4-dioxan-2-one) and lactide including L-lactide, D-lactide and lactic acid (preferably having a mole ratio of p-dioxanone to lactide of from about 40:60 to about 60:40); elastomeric copolymers of epsilon-caprolactone and p-dioxanone (preferably having a mole ratio of epsilon-caprolactone to p- dioxanone of from about 30:70 to about 70:30); elastomeric copolymers of p-dioxanone and trimethylene carbonate (preferably having a mole ratio of p-dioxanone to trimethylene carbonate of from about 30:70 to about 70:30); elastomeric copolymers of trimethylene carbonate and glycolide (preferably having a mole ratio of trimethylene carbonate to glycolide of from about 30:70 to about 70:30); elastomeric copolymer of trimethylene carbonate and lactide including L-lactide, D-lactide, blends thereof or lactic acid copolymers (preferably having a mole ratio of trimethylene carbonate to lactide of from about 30:70 to about 70:30) and blends thereof.

In one embodiment the aliphatic polyester is an elastomeric copolymer of e-caprolactone and glycolide. In another embodiment, the elastomeric copolymers of epsilon-caprolactone and glycolide have a mole ratio of epsilon-caprolactone to glycolide of from about 30:70 to about 70:30. In another embodiment, the elastomeric copolymers of epsilon-caprolactone and glycolide have a mole ratio of epsilon-caprolactone to glycolide of from about 35:65 to about 65:35. In yet another embodiment, the elastomeric copolymers of epsilon-caprolactone and glycolide have a mole ratio of epsilon-caprolactone to glycolide of from about 35:65 to 45:55.

Once a polymer solution has been prepared as described above, the solution is poured into a conventional mold having dimensions such that a thick foam greater than 1 cm may be prepared. The volume of solution added into the mold will depend upon the size of the mold and the desired thickness of the foam. One of skill in the art would be able to determine the appropriate volume of solution to pour into the mold to provide a thick foam of greater than 1 cm based upon the mold size. Optionally, mold inserts may be incorporated into the solution filled mold such that in addition to the uniform porosity and morphological structure of the foam, alternative shapes and contours may be produced and incorporated into the foam, such as channels or pathways.

The mold containing the polymer solution is placed on a precooled shelf in a conventional lyophilizer. The shelf is precooled to a temperature sufficient to effectively induce the solution to form a gel, for example such as a temperature in the range of about 10°C +/- 5°C. Those skilled in the art will appreciate that the temperature will vary with parameters such as polymer concentration and the solvent. The solution is held at the precooled temperature for 360 min to 1440 min such that the solution has completely gelled.

The 1,4-dioxane solvent is then removed from the gelled solution by using an appropriate, conventional lyophilization cycle. For example, after the gelling step as described above, the the lyophilization cycle begins with a freezing step, followed by a primary drying step where vacuum is applied to remove the solvent, lastly multiple secondary drying steps are performed which include slowly increasing the temperature and increasing the vacuum to ensure complete removal of the solvent. Exemplary lyophilization cycles are detailed in the examples below. A thick foam is provided upon completion of the lyophilization cycle. The thick foam has uniform porosity and morphological structure.

Additionally, solids may be added to the polymer-solvent system. The solids added to the polymer-solvent system preferably will not react with the polymer or the solvent. Suitable solids include materials that promote tissue regeneration or regrowth, buffers, reinforcing materials or porosity modifiers. Suitable solids include, but are not limited to, particles of demineralized bone, calcium phosphate particles, or calcium carbonate particles for bone repair, leachable solids for pore creation and particles of biodegradable polymers not soluble in the solvent system as reinforcing or to create pores as they are absorbed. Suitable leachable solids include but are not limited nontoxic leachable materials selected from the group consisting of salts (i.e. sodium chloride, potassium chloride, calcium chloride, sodium tartrate, sodium citrate, and the like) biocompatible mono and disaccharides (i.e. glucose, fructose, dextrose, maltose, lactose and sucrose), polysaccharides (i.e. starch, alginate), water-soluble proteins (i.e. gelatin and agarose). Generally all of these materials will have an average diameter of less than about 1 mm and preferably will have an average diameter of from about 50 to about 500 microns. The particles will generally constitute from about 1 to about 50 volume percent of the total volume of the particle and polymer-solvent mixture (wherein the total volume percent equals 100 weight percent). The leachable materials can be removed by immersing the foam with the leachable material in a solvent in which the particle is soluble for a sufficient amount of time to allow leaching of substantially all of the particles, but which does not dissolve or detrimentally alter the foam. The preferred extraction solvent is water, most preferably distilled-deionized water. Preferably the foam will be dried after the leaching process is complete at low temperature and/or vacuum to minimize hydrolysis of the foam unless accelerated absorption of the foam is desired.

Thick foams made by the method of the present invention having this uniform architecture, as described herein are particularly advantageous in tissue engineering applications to mimic the structure of naturally occurring tissue such as cartilage, skin, bone and vascular tissue. For example by using an elastomeric copolymer of poly(epsilon-caprolactone-co-glycolide) having a molar ratio of (35/65) we can prepare thick elastomeric foams and by using a copolymer of poly(lactide co-glycolide) having a molar ratio of 95/5 we can prepare thick foams that are hard and stiff. A foam may be formed that transitions from a softer spongy foam to a stiffer more rigid foam similar to the transition from cartilage to bone by preparing thick foams from blends of the poly(epsilon-caprolactone-co-glycolide) having a molar ratio of (35/65) and a copolymer of poly(lactide co-glycolide) having a molar ratio of 95/5. Clearly other polymer blends may be used for similar gradient effects or to provide different gradients such as different absorption profiles, stress response profiles, or different degrees of elasticity.

The novel thick foams made by the method of the present invention manufactured by the novel processes of the present invention are useful in the preparation of medical devices such as tissue scaffolds for applications such skin regeneration and cartilage regeneration. The foams may also be used in combination with other devices that can be added during the lyophilization step. For e.g. meshes and nonwovens. Also foams made using this process and using materials such as 95/5 PLA/PGA, are stiff and strongThe thick foams of the present invention may be further processed to prepare medical devices. The thick foams may be machined or laser cut, or processed using other conventional techniques, to provide medical devices and components of medical devices including but not limited to thin foam sheets or films, three-dimensional devices having symmetrical or asymmetrical shapes or structures, including screws, pins, implants, mesh-like implants, etc., and three-dimensional asymmetrically shaped structures, such as irregular shapes or structures for organ tissue engineering and contoured to fit irregular tissue defects, such as bone or soft tissue.

The following examples are illustrative of the principles and practice of this invention, although not limited thereto. Numerous additional embodiments within the scope of the invention as defined in the accompanying claims will become apparent to those skilled in the art once having the benefit of this disclosure.

### Example 1:

This example describes the preparation of thick foams for tissue implants. A thermoreversible polymer solution was prepared. A 90/10-weight ratio solution of 1,4 dioxane/(35/65 polycaprolactone/polyglycolide) (PCL/PGA), (Ethicon, Inc., Somerville, NJ) was weighed into a flask. The flask was placed in a water bath, with stirring at 70°C for 5 -6 hours. The solution was then filtered using an extraction thimble, extra coarse porosity, type ASTM 170-220 (EC) and stored in flask at room temperature.

A Kinetics thermal system (FTS Dura Freeze Dryer) (Model # TD3B2T5100): Stone Ridge, NY) was used to carry out the experiment. The shelf was pre-cooled to a temperature of 12 °C. The polymer solution prepared above was poured into a 4.5in. x 4.5in. x 2.5in mold (for a 2.5 cm foam 330 mL of the solution was used). The mold was a rectangular trough made of aluminum and coated with Teflon. The solution filled mold was placed on the precooled shelf. The cycle was run using the conditions from Table 1.

**Table 1: Freeze Drying conditions for Example 2**

| Steps | Temperature (°C) | Rate (°C/min) | Time (min) | Vacuum (mTorr) |
|---|---|---|---|---|
| Gel Step | 12 | 2.5 | 1440 | no vacuum |
| Freezing Step | -17 | 0.1 | 15 | no vacuum |
| Primary Drying | -17 | 2.5 | 600 | 1000 |
| Secondary Drying - 1 | -7 | 2.5 | 300 | 100 |
| Secondary Drying - 2 | 5 | 2.5 | 300 | 100 |
| Secondary Drying - 3 | 20 | 2.5 | 150 | 100 |
| Secondary Drying - 4 | 30 | 2.5 | 150 | 100 |

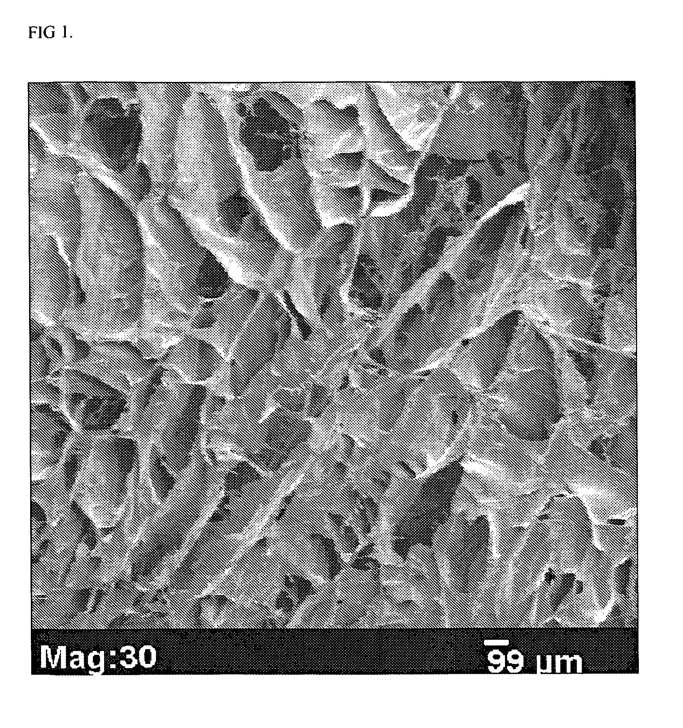

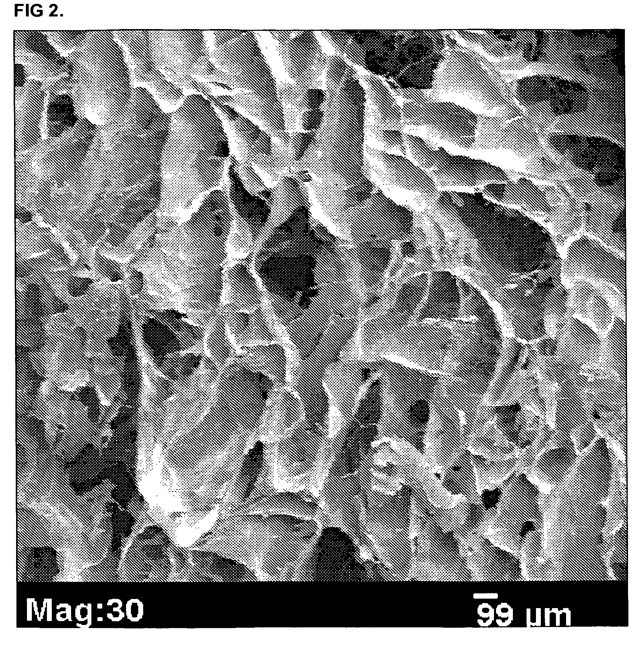

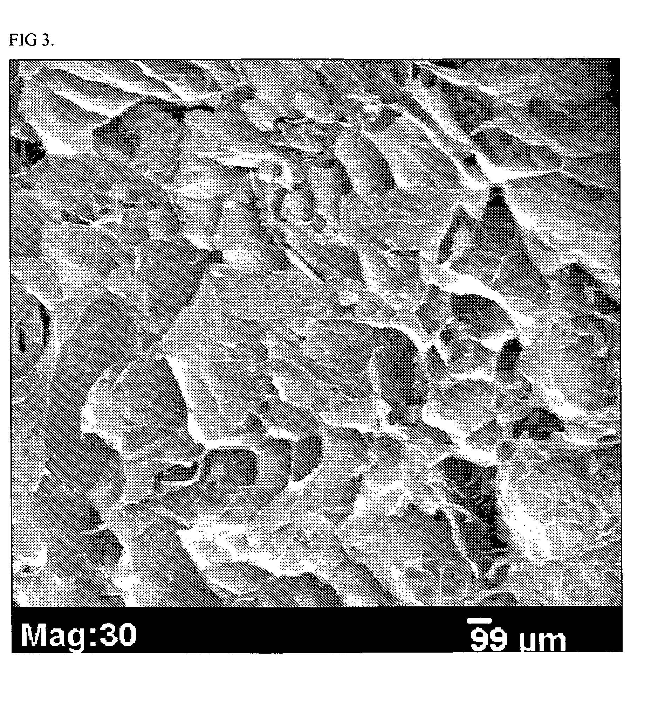

In the gelling step the shelves were maintained at a temperature of 12°C for 1440 min. during which time the solution was allowed to gel. The shelf temperature was set to -17°C for 15 min. at cooling ramp rate of 0.1 °C/min for the freezing step. At the end of this step, the temperature was held at -17°C for 250 min, to make sure that the gelled solution is completely frozen. At the end of the freezing cycle, the drying step was initiated for the sublimation of 1, 4 dioxane. In the first step vacuum was applied at 1000 mTorr, keeping the shelf temperature at -17°C. These conditions were set for 600 min. The secondary drying was carried in four steps, to remove any residual dioxane. First the temperature was raised to - 7°C at heating ramp rate of 2.5°C/min and held for 300 min at a vacuum was set to 100 mTorr. The temperature was then raised to 5 °C and held for 300 min at vacuum level of 100 mTorr. In the third stage the temperature was then raised to 20°C for 150 min at a vacuum level of 100. In the final stage of the secondary drying step, the lyophilizer was brought to room temperature and held for 150min and 100 mTorr. At the end of this step, the cycle was stopped and the vacuum broken. The thick foam was taken out of the mold and samples were provided for scanning electron microscopy (SEM). FIGS. 1, 2, and 3 shows the SEM images for the bottom, middle and top cross-sections of the thick foam sample. The SEM images showed that uniform porosity was achieved throughout the cross section of the scaffold. The final thickness obtained after lyophilization was about 2.2 cm. The pore architecture was uniform in terms of its morphology and pore size throughout the thickness of the foam structure.

### Example 2

A thermoreversible polymer solution was prepared from 35/65 PCL/PGA and 1,4-dioxane as described in Example 1.

A 4.5in. x 4.5in. x 2.5in. mold (aluminum mold coated with Teflon) was filled with 330 ml the polymer solution to prepare a foam of about 2.5 cm in thickness and was placed on the freeze dryer shelf (FTS Dura Freeze Dryer) that was precooled to a temperature of 12°C. Table 2 describes the lyophilization steps. In this experiment, in the lst step of the drying cycle the temperature of the shelf was lowered to -17°C at slow ramp rate of 0.1°C/min.

**Table 2: Freeze drying conditions for Example 2**

| Steps | Temperature (°C) | Rate (°C/min) | Time (min) | Vacuum (mTorr) |
|---|---|---|---|---|
| Gel Step | 12 | 2.5 | 1440 | no vaccuum |
| Freezing Step 1 | -17 | 0.1 | 15 | no vaccuum |
| Freezing Step 1 | -15 | 0.1 | 250 | no vaccuum |
| Primary Drying | -17 | 2.5 | 600 | 1000 |
| Secondary Drying - 1 | -7 | 2.5 | 300 | 100 |
| Secondary Drying - 2 | 5 | 2.5 | 300 | 100 |
| Secondary Drying - 3 | 20 | 2.5 | 150 | 100 |
| Secondary Drying - 4 | 30 | 2.5 | 150 | 100 |

The thick dry foam was removed from the mold. A sample was cut from this foam for analysis by SEM in order to evaluate the pores. The SEM images for the top, middle and the bottom surface were taken. The SEM images again showed uniform pore morphology similar to the thick foam prepared in Example 1.

### Example 3

A thermoreversible polymer solution was prepared using 95/5 poly(lactide-co-glycolide)(PLA/PGA) and 1,4-dioxane according to the methods of Example 1. A 4.5in. x 4.5in. x 2.5in mold (aluminum mold coated with Teflon) was filled with 330ml the polymer solution to prepare a foam of about 2.5 cm in thickness and was placed on the freeze dryer shelf (FTS Dura Freeze Dryer) that was precooled to a temperature of 12°C. Table 4 describes the lyophilization cycle. In this experiment, in the 2^{nd} step of the freezing cycle the temperature of the shelf was lowered to -17°C at slow ramp rate of 0.1°C/min.

**Table 4: Freeze drying conditions for Example 4**

| Steps | Temperature (°C) | Rate (°C/min) | Time (min) | Vacuum (mTorr) |
|---|---|---|---|---|
| Gel Step | 12 | 2.5 | 1440 | no vaccuum |
| Freezing Step 1 | -17 | 0.1 | 15 | no vaccuum |
| Freezing Step 1 | -15 | 0.1 | 250 | no vaccuum |
| Primary Drying | -17 | 2.5 | 600 | 1000 |
| Secondary Drying - 1 | -7 | 2.5 | 300 | 100 |
| Secondary Drying - 2 | 5 | 2.5 | 300 | 100 |
| Secondary Drying - 3 | 20 | 2.5 | 150 | 100 |
| Secondary Drying - 4 | 30 | 2.5 | 150 | 100 |

The thick dry foam was removed from the mold. A sample was cut from this foam for SEM characterization in order to evaluate the pores. The SEM images for the top, middle and the bottom cross-sections were taken for the scaffold. The foam morphology was again similar to the foam prepared in Example 1.

### Example 4

A thermoreversible polymer solution was prepared from 35/65 PCL/PGA and 1,4-dioxane as described in Example 1.A 2in. x 2in. x 3/4in. mold (aluminum mold coated with teflon) was filled with 330 ml the polymer solution to prepare a foam about 1 cm in thickness and one-millimeter diameter telfon coated pins were inserted into an aluminum top mold in a regular array (3X5). The spacing between the pins was 2mm.

The solution filled mold was placed on the freeze dryer shelf (FTS Dura Freeze Dryer) that was precooled to a temperature of 12°C. Table 5 describes the lyophilization steps cycle. In this experiment, in the 2^{nd} step of the freezing cycle the temperature of the shelf was lowered to -17°C at slow ramp rate of 0.1°C/min.

**Table 5: Freeze drying conditions for Example 5**

| Steps | Temperature (°C) | Rate (°C/min) | Time (min) | Vacuum (mTorr) |
|---|---|---|---|---|
| Gel Step | 12 | 2.5 | 1440 | no vaccuum |
| Freezing Step 1 | -17 | 0.1 | 15 | no vaccuum |
| Freezing Step 1 | -15 | 0.1 | 250 | no vaccuum |
| Primary Drying | -17 | 2.5 | 600 | 1000 |
| Secondary Drying - 1 | -7 | 2.5 | 300 | 100 |
| Secondary Drying - 2 | 5 | 2.5 | 300 | 100 |
| Secondary Drying - 3 | 20 | 2.5 | 150 | 100 |
| Secondary Drying - 4 | 30 | 2.5 | 150 | 100 |

The thick, dry foam was removed from the mold. Similarly, foams greater than 1cm in thickness may be prepared using mold inserts to create various foam shapes and contours, as well as secondary foam structures including channels and the like.

## Claims

1. A method of making a polymer foam member having a thickness of greater than 1 cm, comprising the steps of:
providing a thermoreversible polymer solution, said solution comprising a biocompatible, biodegradable polymer and a solvent;
placing said solution in a mold;
cooling the solution in said mold to a precool temperature and holding the solution at said precool temperature for from 360 minutes to 1440 minutes until the solution gels; followed by
removing the solvent by lyophilization to yield said foam member having a thickness of greater than 1 cm and inter-connected pores.

2. The method of claim 1,wherein the biocompatible, biodegradable polymer comprises a polymer selected from the group consisting of aliphatic polyester is selected from the group consisting of homopolymers and copolymers of lactide, lactic acid, glycolide, glycolic acid), ε-caprolactone, p-dioxanone (1,4-dioxan-2-one), trimethylene carbonate (1,3-dioxan-2-one), alkyl derivatives of trimethylene carbonate, δ-valerolactone, β-butyrolactone, γ-butyrolactone, ε-decalactone, hydroxybutyrate, hydroxyvalerate, 1,4-dioxepan-2-one, 1,5,8, 12-tetraoxacyclotetradecane-7,14-dione), 1,5-dioxepan-2-one, 6,6-dimethyl-1,4-dioxan-2-one and polymer blends thereof.

3. The method of claim 1, wherein the solvent is 1,4-dioxane.

4. The method of claim 1, wherein the lyophilization comprises a first freezing step and first drying step, and at least one subsequent additional drying step.

5. The method of claim 1, wherein the solution additionally comprises a leachable solid selected from the group consisting of sodium chloride, potassium chloride, calcium chloride, sodium tartrate, sodium citrate, glucose, fructose, dextrose, maltose, lactose, sucrose and combinations thereof.

6. The method of claim 1, wherein the solution additionally comprises a therapeutic agent selected from the group consisting consisting of antiinfectives, hormones, analgesics, anti-inflammatory agents, growth factors, chemotherapeutic agents, antirejection agents, prostaglandins, RDG peptides and combinations thereof.

7. The method of claim 1, wherein the pores have a uniform morphology such that the pore size ranges are uniform through the thickness of the scaffold.

8. A method of making a medical device comprising the steps of:
making a foam member by a method according to any of claims 1 to 8; and
cutting the foam member into a medical device.

## Patentansprüche

1. Verfahren zur Herstellung eines Polymerschaumstoffelements mit einer Dicke von mehr als 1 cm, bei dem man:
eine thermoreversible Polymerlösung, die ein biokompatibles, biologisch abbaubares Polymer und
ein Lösungsmittel umfasst, bereitstellt;
die Lösung in eine Form einbringt;
die Lösung in der Form auf eine Vorkühltemperatur abkühlt und die Lösung 360 Minuten bis 1440 Minuten bei der Vorkühltemperatur hält, bis die Lösung geliert; und dann
das Lösungsmittel durch Gefriertrocknung entfernt,
wobei man das Schaumstoffelement mit einer Dicke von mehr als 1 cm und miteinander verbundenen Poren erhält.

2. Verfahren nach Anspruch 1, bei dem das biokompatible, biologisch abbaubare Polymer ein Polymer aus der Gruppe bestehend aus aliphatischem Polyester aus der Gruppe bestehend aus Homopolymeren und Copolymeren von Lactid, Milchsäure, Glykolid, Glykolsäure, ε-Caprolacton, p-Dioxanon (1,4-Dioxan-2-on), Trimethylencarbonat (1,3-Dioxan-2-on), Alkylderivaten von Trimethylencarbonat, δ-Valerolacton, β-Butyrolacton, γ-Butyrolacton, ε-Decalacton, Hydroxybutyrat, Hydroxyvalerat, 1,4-Dioxepan-2-on, 1,5,8,12-Tetraoxacyclotetradecan-7,14-dion,1,5-Dioxepan-2-on, 6,6-Dimethyl-1,4-dioxan-2-on und Polymermischungen davon umfasst.

3. Verfahren nach Anspruch 1, bei dem es sich bei dem Lösungsmittel um 1,4-Dioxan handelt.

4. Verfahren nach Anspruch 1, bei dem die Gefriertrocknung einen ersten Gefrierschritt und einen ersten Trocknungsschritt und mindestens einen nachfolgenden Trocknungsschritt umfasst.

5. Verfahren nach Anspruch 1, bei dem die Lösung zusätzlich einen auslaugbaren Feststoff aus der Gruppe bestehend aus Natriumchlorid, Kaliumchlorid, Calciumchlorid, Natriumtartrat, Natriumcitrat, Glucose, Fructose, Dextrose, Maltose, Lactose, Saccharose und Kombinationen davon umfasst.

6. Verfahren nach Anspruch 1, bei dem die Lösung zusätzlich ein therapeutisches Mittel aus der Gruppe bestehend aus Antiinfektiva, Hormonen, Analgetika, Antiphlogistika, Wachstumsfaktoren, Chemotherapeutika, Antiabstoßungsmitteln, Prostaglandinen, RDG-Peptiden und Kombinationen davon umfasst.

7. Verfahren nach Anspruch 1, bei dem die Poren eine einheitliche Morphologie aufweisen, so dass die Porengrößenbereiche über die gesamte Dicke des Scaffolds einheitlich sind.

8. Verfahren zur Herstellung einer medizinischen Vorrichtung, bei dem man:
nach einer Methode gemäß einem der Ansprüche 1 bis 7 ein Schaumstoffelement herstellt und
das Schaumstoffelement zu einer medizinischen Vorrichtung schneidet.

## Revendications

1. Méthode de fabrication d'un élément de mousse de polymère ayant une épaisseur supérieure à 1 cm, comprenant les étapes consistant à :
fournir une solution de polymère thermoréversible, ladite solution comprenant un polymère biocompatible et biodégradable, et un solvant ;
placer ladite solution dans un moule ;
refroidir la solution dans ledit moule jusqu'à une température de pré-refroidissement et maintenir la solution à ladite température de pré-refroidissement pendant de 360 minutes à 1440 minutes jusqu'à gélification de la solution ; suivie par l'étape consistant à
éliminer le solvant par lyophilisation pour conduire audit élément de mousse ayant une épaisseur supérieure à 1 cm et des pores interconnectés.

2. Méthode selon la revendication 1, dans laquelle le polymère biocompatible et biodégradable comprend un polymère choisi dans le groupe constitué par un polymère aliphatique choisi dans le groupe constitué par des homopolymères et copolymères de lactide (acide lactique), de glycolide (acide glycolique), d'ε-caprolactone, de p-dioxanone (1,4-dioxan-2-one), de carbonate de triméthylène (1,3-dioxan-2-one), de dérivés alkyle du carbonate de triméthylène, de δ-valérolactone, de β-butyrolactone, de γ-butyrolactone, d'ε-décalactone, d'hydroxybutyrate, d'hydroxyvalérate, de 1,4-dioxépan-2-one, de 1,5,8,12-tétraoxacyclo-tétradécane-7,14-dione, de 1,5-dioxépan-2-one, de 6,6-diméthyl-1,4-dioxan-2-one, et des mélanges polymères de ceux-ci.

3. Méthode selon la revendication 1, dans laquelle le solvant est le 1,4-dioxane.

4. Méthode selon la revendication 1, dans laquelle la lyophilisation comprend une première étape de congélation et une première étape de séchage, et au moins une étape de séchage supplémentaire ultérieure.

5. Méthode selon la revendication 1, dans laquelle la solution comprend en outre un solide lixiviable choisi dans le groupe constitué par le chlorure de sodium, le chlorure de potassium, le chlorure de calcium, le tartrate de sodium, le citrate de sodium, le glucose, le fructose, le dextrose, le maltose, le lactose, le saccharose, et des combinaisons de ceux-ci.

6. Méthode selon la revendication 1, dans laquelle la solution comprend en outre un agent thérapeutique choisi dans le groupe constitué par les agents anti-infectieux, les hormones, les analgésiques, les agents anti-inflammatoires, les facteurs de croissance, les agents chimiothérapeutiques, les agents antirejet, les prostaglandines, les peptides RDG, et des combinaisons de ceux-ci.

7. Méthode selon la revendication 1, dans laquelle les pores possèdent une morphologie uniforme telle que les domaines de taille de pores sont uniformes dans l'ensemble de l'épaisseur de l'échafaudage.

8. Méthode de fabrication d'un dispositif médical, comprenant les étapes consistant à :
fabriquer un élément de mousse par une méthode selon l'une quelconque des revendications 1 à 7 ; et
découper l'élément de mousse en un dispositif médical.
